# EUROPEAN PATENT APPLICATION

(11) **EP 1 930 443 A1**
(43) Date of publication of application: **11.06.2008**
(21) Application number: 06798475.7
(22) Date of filing: 29.09.2006
(51) Int. Cl.: C12Q 1/60, C12Q 1/26, C12Q 1/61, G01N 21/78, G01N 33/52, G01N 33/92

(54) **METHOD FOR SELECTIVE, SIMULTANEOUS QUANTIFICATION OF TWO SUBSTANCES IN BIOLOGICAL SAMPLE**

(30) Priority: 30.09.2005 JP 2005288689
(71) Applicant: DENKA SEIKEN Co., Ltd., Chuo-ku, Tokyo 103-0025 (JP)
(72) Inventor: ITOH, Yasuki, Oaza Kigoshi Gosen-shi, Niigata 959-1834 (JP); MATSUMOTO, Keiko, Oaza Kigoshi Gosen-shi, Niigata 959-1834 (JP); MATSUI, Hiroshi, Oaza Kigoshi Gosen-shi, Niigata 959-1834 (JP); HIRAO, Yuhko, Oaza Kigoshi Gosen-shi Niigata 959-1834 (JP)
(74) Representative: HOFFMANN EITLE
(86) International application number: PCT/JP2006/319557
(87) International publication number: WO 2007/037419

(57) **Abstract**

This invention provides a method for differentially and simultaneously quantifying two target analytes via a single assay operation with the use of a single type of reagent in each of the two steps, i.e., the first step and the second step. In this method, the reaction product associated with the first target analyte is detected at an early stage of the second step and the reaction product associated with the second target analyte is then detected.

## Description

### Background Art

Clinical testing is employed for analysis, diagnosis, identification of prognosis, or the like with respect to pathologic conditions. The importance of clinical testing for the purpose of accurate examination of patient conditions is increasing. The number of test items and the number of specimens used are continuously rising. Meanwhile, increase in overall national medical expenditure has been a serious object of public concern, and a reduction of such expenditure has been strongly desired. Many clinical testing techniques involve the use of automated analyzers, and the development of rapid and simple testing, methods and reagents for testing many items with the use of small specimen quantities, and the like have been expected. With the provision of such reagents, many people can receive necessary clinical testing as the need arises, which in turn leads to more accurate treatment and prevention. For example, testing of many items with respect to blood cholesterol in a rapid and simple manner has been desired.

Cholesterol elevation has been assayed for a long time as a factor that develops and advances arteriosclerosis in the vascular endothelium and increases the risk of coronary artery disease. Also, triglyceride has been heretofore used as a marker of diabetics or postprandial hyperlipidemia, and it has again drawn attention as a metabolic syndrome risk factor in recent years.

Such lipid components are present in the form of lipoproteins in the blood, and lipoproteins are classified as chylomicron (CM), very low density lipoprotein (VLDL), LDL, HDL, and the like. LDL elevation causes arteriosclerotic diseases and HDL has a function of suppressing the same.

Up to the present, HDL cholesterol assay techniques that do not require isolation have been reported and extensively employed. LDL cholesterol has commonly been calculated by the Friedewald formula; however, an assay technique that does not require fractionation has been reported in recent years (see Patent Document 1). Also, a method for assaying triglyceride value selectively in HDL or in LDL has been reported. At present, assay for total cholesterol and HDL cholesterol have been extensively used; however, LDL cholesterol is still primarily determined using a formula, and an assay technique that does not require fractionation has not yet prevailed.

Further, methods for continuously and differentially assaying two types of cholesterol values via a single assay operation have been reported (see Patent Documents 1 and 2). These techniques, however, involve the use of three types of reagents for assaying two types of cholesterols in three different steps. Accordingly, an apparatus that can be employed is limited. Also, methods for continuously and differentially assaying two types of cholesterol values with the use of two types of reagents have been reported (see Patent Documents 4 and 5). In these techniques, different types of cholesterols are allowed to react independently in the first step and in the second step, and the absorbance is then measured. In such technique, however, the first reagent is capable of generating a quinone pigment in the liquid state at the time of use. Because of this, the reagent disadvantageously undergoes air oxidation and develops color spontaneously. Thus, such reagent had been considered to be unstable as a liquid reagent.
Patent Document 1: JP Patent Publication (kokai) No. 11-318496 (A) (1999)
Patent Document 2: JP Patent Publication (kohyo) No. 2003-501630 (A)
Patent Document 3: JP Patent Publication (kokai) No. 2001-124780 (A)
Patent Document 4: WO 04/055204
Patent Document 5: WO 00/17388

### Disclosure of the Invention

### Objects to be attained by the Invention

The present invention provides a differential quantification method wherein two target analytes are simultaneously quantified at one time via a single assay operation with using respective reagents in both two steps consisting of the first step and the second step. Examples of the two target analytes include two types of lipoprotein components having different properties, such as LDL cholesterol and total cholesterol. The present invention also provides a method for stabilizing a reagent used for simultaneously and differentially quantifying the two target analytes via a single assay operation while suppressing spontaneous color development.

### Means for Attaining the Objects

The present inventors have conducted concentrated studies concerning a method for assaying cholesterol, triglyceride, and the like, which simultaneously quantifies two components among such components contained in total, LDL, and HDL in the blood. As a result, they succeeded in developing a quantification method that realizes long-term stability, even if a reagent is in a liquid state.

Specifically, the procedure from reaction to detection was carried out in the first step in the aforementioned simultaneous assay method using two types of reagents only (WO 04/055204); however, the present invention has enabled detection of the reaction that had occurred in the first step at an early stage in the second step and the detection of the other analyte thereafter.

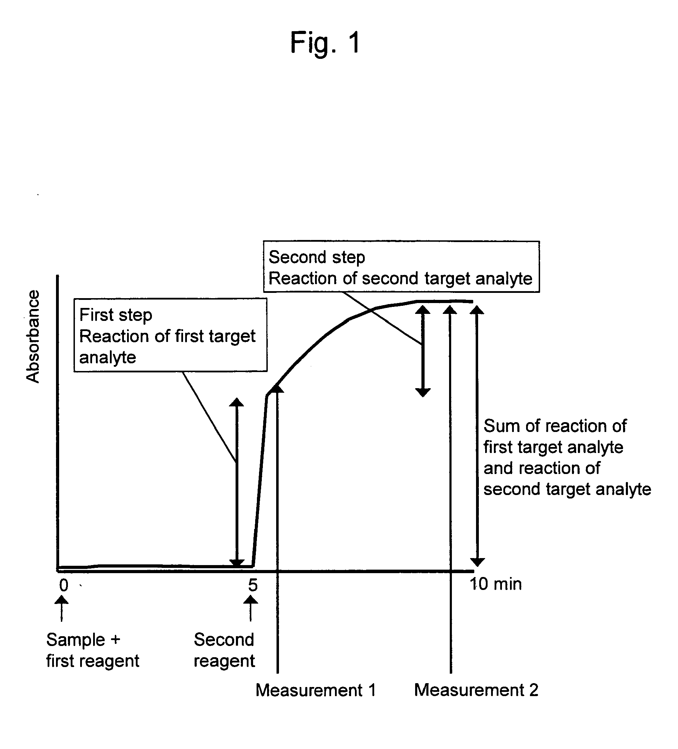

Fig. 1 shows the principle of simultaneous and differential quantification according to the present invention. As shown in Fig. 1, the method of the present invention comprises two steps. In the first step, a sample is mixed with a first reagent to cause the reaction of the first target analyte, and the reaction product is then generated. The resulting reaction product is detected at an early stage of the second step (measurement 1). In the second step, a second reagent is added to further cause the reaction of the second target analyte, and the reaction product is then generated. In measurement 2, the reaction product of the first step and the reaction product of the second step are detected (measurement 2). When two lipoprotein components (e.g., HDL cholesterol and LDL cholesterol) are supplied to target analytes, hydrogen peroxide is generated by the first target-analyte-based reaction in the first step, the hydrogen peroxide generated in the first step changes the absorbance of the reaction solution in the second step, then the second target-analyte-based reaction takes place, and changes in the absorbance of the reaction solution due to such reaction are assayed. The total extent of change in the absorbance in the second step is equivalent to the sum of the amount the first target analyte and those of the second target analyte, and the extent of change in the absorbance relative to the amount of hydrogen peroxide generated in the second step is equivalent to the amount of the second target analyte. The parameters for analyzing the changes in the absorbance using an automated analyzer may be changed to simultaneously measure two values via a single assay operation. This enables simultaneous quantification of two target analytes. When target analytes are one lipid component and the total amount of lipid components (e.g., LDL cholesterol and total cholesterol) in the given lipoprotein, hydrogen peroxide is generated by the reaction based on lipoproteins (e.g., cholesterol in CM, VLDL, or HDL) other than the given lipoprotein in the first step, the hydrogen peroxide generated in the first step changes the absorbance of the reaction solution in the second step, the reaction based on the given lipoprotein (e.g., cholesterol in LDL) takes place, and then changes in the absorbance of the reaction solution due to such reaction are measured. The extent of change in the absorbance throughout the reaction corresponds to the total amount of lipid components, and the extent of change in the absorbance relative to the amount of hydrogen peroxide generated in the second step is equivalent to the amount of the given lipoprotein (e.g., cholesterol in LDL).

According to conventional simultaneous and differential quantification techniques, a plurality of reagent compositions involved in generation of a quinone pigment would be intensively contained in the first reagent used in the first step of assay. According to the method of the present invention, a quinone pigment is generated only in the second step, and a plurality of reagent compositions involved in generation of a quinone pigment can be separated into the first reagent used in the first step and the second reagent used in the second step. This enables suppression of spontaneous color development due to air oxidation of the reagent. Thus, reagents can be stabilized and the reagents became capable of use for assaying target analytes stably.

When the method of the present invention is performed using an automated analyzer in which various assay parameters can be set, the total amount of a given lipid component, such as total cholesterol, or the sum of two target analytes, such as LDL cholesterol and HDL cholesterol, is quantified based on the total extent of change in the absorbance in the second step, under an assay parameter for analyzing multiple items with an automated analyzer. Also, a target analyte can be quantified based on the extent of change in the absorbance between two time points after the addition of the second reagent in the second step (i.e., after the rapid changes in the absorbance immediately after the addition of the second reagent and at the termination point of the reaction). When the measured value, which is equivalent to the sum of the two target analytes, is calculated based on the total extent of change in the absorbance in the second step, the measured value of a target analyte obtained by the extent of change in the absorbance between two time points after the addition of the second reagent may be subtracted to determine the measured value for the other target analyte.

Further, the method of the present invention enables simultaneous quantification of two target analytes in a sample, without the limitation of target analytes.

Specifically, the present invention is as follows.
[1] A method for simultaneously quantifying two target analytes in a sample using two types of reagents, wherein the reaction product associated with the first target analyte is detected at an early stage of the second step and then the reaction product associated with the second target analyte is detected.
[2] The method for simultaneously quantifying two target analytes in a sample using two types of reagents according to [1] above, wherein the first target analyte reacts with a reagent composition contained in the first reagent to generate the first reaction intermediate, the second target analyte reacts with a reagent composition contained in the second reagent to generate the second reaction intermediate, the first reaction intermediate reacts with the reagent composition contained in the second reagent to generate the optically measurable first reaction product, the second reaction intermediate reacts with the reagent composition contained in the second reagent to generate the optically measurable second reaction product, the method comprising the first step of adding the first reagent to the sample to treat the first target analyte and generate the first reaction intermediate and the second step of adding the second reagent to generate the first reaction product from the first reaction intermediate obtained in the first step and treating the second target analyte to generate the second reaction product from the resulting second reaction intermediate, wherein, in the first step, the first reaction product is not generated and, in the second step, measuremen is carried out twice, i.e., the first reaction product originating from the first target analyte is measured in the first measurement, and the second reaction product originating from the second target analyte or the first reaction product originating from the first target analyte and the second reaction product originating from the second target analyte are measured in the second measurement, thereby simultaneously quantifying two target analytes based on the amounts of the resulting first and second reaction products.
[3] The method according to [1] or [2] above, wherein the first reaction product originating from the first target analyte and the second reaction product originating from the second target analyte have the same absorption wavelengths.
[4] The method according to [3] above, wherein the first reaction product originating from the first target analyte and the second reaction product originating from the second target analyte are the same substances.
[5] The method according to any of [1] to [4] above, wherein the first reaction intermediate and the second reaction intermediate are the same substances.
[6] The method according to any of [1] to [5] above, wherein the first target analyte and the second target analyte are selected from the group consisting of lipid components in the analyte sample, i.e., creatinine, uric acid, glucose, glutamate oxaloacetate transaminase (GOT) (aspartate aminotransferase (AST)), glutamate pyruvate transaminase (GPT) (alanine aminotransferase (ALT)), γ-glutamyl transpeptidase (γ-GTP), lactate dehydrogenase (LDH), alkaline phosphatase (ALP), creatine phosphokinase (CPK), and amylase (AMY).
[7] The method according to [6] above, wherein the first target analyte and the second target analyte are lipid components in the analyte sample.
[8] The method according to [7] above, wherein the lipid components are cholesterol or triglyceride components in the lipoprotein.
[9] The method according to any of [1] to [8] above, wherein the first and the second reaction products originating from the first and the second target analytes and the first and the second reaction products originating from the first and the second reaction intermediates are generated by oxidation-reduction reaction.
[10] The method for simultaneously quantifying two lipid components in a sample using two types of reagents according to any of [1] to [9] above, which comprises the first step of treating the first target analyte in a biological sample to generate hydrogen peroxide and the second step of converting hydrogen peroxide obtained in the first step into a quinone pigment and treating the second target analyte to convert the resulting hydrogen peroxide into a quinone pigment, wherein a quinone pigment is not generated in the first step, and the second step comprises two measurements of the first measurement of a quinone pigment originating from the first target analyte and the second measurement of quinone pigments originating from the first target analyte and the second target analyte, thereby simultaneously quantifying two target analytes based on the amounts of the generated quinone pigments.
[11] The method according to [10] above, wherein the reagent composition associated with generation of a quinone pigment comprises 4-aminoantipyrine, a phenol or aniline hydrogen donor compound, and peroxidase and the first step involves the addition of either a 4-aminoantipyrine or a phenol or aniline hydrogen donor compound and the second step involves the addition of a reagent composition that was not added in the first step.
[12] The method according to [10] or [11] above, wherein the assay of the lipid component is an assay of a cholesterol or triglyceride component in the lipoprotein.
[13] The method according to any of [10] to [12] above, wherein, when the lipid component is cholesterol, the two target analytes are total cholesterol and low-density lipoprotein (hereafter referred to as "LDL") cholesterol, total cholesterol and high-density lipoprotein (hereafter referred to as "HDL") cholesterol, or LDL cholesterol and HDL cholesterol in the blood.
[14] The method according to any of [10] to [12] above, wherein, when the lipid component is triglyceride, two target analytes are total triglyceride and LDL triglyceride, total triglyceride and HDL triglyceride, or LDL triglyceride and HDL triglyceride in the blood.
[15] The method according to any of [10] to [14] above, wherein, when the two target analytes are total lipid components and a lipid component in HDL or LDL, the method comprises the first step of generating hydrogen peroxide in the presence of a reagent comprising an enzyme and a surfactant that selectively act on lipoproteins other than HDL or LDL and the second step of converting hydrogen peroxide obtained in the first step into a quinone pigment and generating hydrogen peroxide in the presence of a reagent that selectively reacts with HDL or LDL to convert hydrogen peroxide into a quinone pigment.
[16] The method according to any of [10] to [14] above, which, when the two target analytes are lipid components in HDL and LDL, comprises the first step of generating hydrogen peroxide in the presence of a reagent comprising a given enzyme and surfactant that selectively act on HDL and the second step of converting hydrogen peroxide obtained in the first step into a quinone pigment and generating hydrogen peroxide in the presence of a reagent that selectively reacts with LDL to convert hydrogen peroxide into a quinone pigment.
[17] The method according to any of [1] to [16] above, wherein, changes in the absorbance in the second step indicate a two-phase increase comprising a rapid increase in the absorbance immediately after the addition of the second reagent and a mild increase thereafter, and a target analyte is quantified based on the amount of the latter mild change in the absorbance.
[18] The method according to any of [1] to [17] above, wherein the total cholesterol is quantified based on the total extent of change in the absorbance in the second step.
[19] The method according to any of [1] to [18] above, wherein the analysis is carried out using an automated clinical biochemical analyzer under different assay parameters via a single assay operation.
[20] The method according to any of [1] to [19] above, wherein the first and the second steps each comprise the addition of respective liquid reagents.
[21] A method for stabilizing a liquid reagent in the method according to any of [1] to [20] above, wherein the first reagent that is added in the first step comprises a reagent composition associated with generation of a quinone pigment, i.e., any of 4-aminoantipyrine and a phenol or aniline hydrogen donor compound, and the second reagent comprises a substance that is not contained in the first reagent selected from among 4-aminoantipyrine, a phenol or aniline hydrogen donor compound, and peroxidase.
[22] A kit for performing a method for simultaneously quantifying two lipid components in a sample using two types of reagents, the method comprising the first step of treating the first target analyte in a biological sample to generate hydrogen peroxide and the second step of converting hydrogen peroxide obtained in the first step into a quinone pigment and treating the second target analyte to convert the resulting hydrogen peroxide into a quinone pigment, wherein a quinone pigment is not generated in the first step and the second step comprises two measure operations of the first measurement of a quinone pigment originating from the first target analyte and the second measurement of quinone pigments originating from the first target analyte and the second target analyte, thereby simultaneously quantifying two target analytes based on the amounts of generated quinone pigments, and wherein the first reagent comprises a reagent composition associated with generation of a quinone pigment, i.e., any of 4-aminoantipyrine and a phenol or aniline hydrogen donor compound, and the second reagent comprises a substance that is not contained in the first reagent selected from among 4-aminoantipyrine, a phenol or aniline hydrogen donor compound, and peroxidase.
[23] The kit according to [22] above, wherein the first reagent comprises a surfactant and an enzyme that act on the first target analyte, and the second reagent at least comprises a surfactant that acts on the second target analyte.

### Effects of the Invention

The method of the present invention enables simultaneous quantification of different two substances via a single assay operation. Further, reagents to be used do not spontaneously develop color due to air oxidation, and such reagents can be maintained stably in a liquid state.

This description includes part or all of the contents as disclosed in the description and/or drawings of Japanese Patent Application No. 2005-288689, which is a priority document of the present application.

### Brief Description of the Drawings

Fig. 1 presents the principle of the simultaneous and differential quantification method of the present invention.
Fig. 2 shows changes in the absorption spectra for reagents used for simultaneous assay of LDL cholesterol and total cholesterol due to storage of reagents in the reagent system 1, which is a control sample comprising all coloring agents in the first reagent.
Fig. 3 shows changes in the absorption spectra for reagents used for simultaneous assay of LDL cholesterol and total cholesterol due to storage of reagents in the reagent system 2 of the present invention.
Fig. 4 shows changes in the calibration absorption of total cholesterol assayed with the use of a control reagent.
Fig. 5 shows changes in the calibration absorption of total cholesterol assayed according to the present invention.
Fig. 6 shows a correlation between the LDL cholesterol value assayed using the reagents for simultaneously assaying LDL cholesterol and total cholesterol and the LDL cholesterol value assayed in a reagent solely using LDL cholesterol.
Fig. 7 shows a correlation between the total cholesterol value assayed using the reagents for simultaneously assaying LDL cholesterol and total cholesterol and the total cholesterol value assayed, in a reagent used solely for using assaying total cholesterol.

### Best Modes for Carrying out the Invention

The present invention relates to a method for simultaneously quantyfying two target analytes in a biological sample as the analyte sample via a single assay operation. Examples of target analytes include, but are not limited to, lipid components such as cholesterol and triglyceride, creatinine, uric acid, glucose, glutamate oxaloacetate transaminase (GOT) (aspartate aminotransferase (AST)), glutamate pyruvate transaminase (GPT) (alanine aminotransferase (ALT)), γ-glutamyl transpeptidase (γ-GTP), lactate dehydrogenase (LDH), alkaline phosphatase (ALP), creatine phosphokinase (CPK), and amylase (AMY). As cholesterol or triglyceride, one in the lipoprotein may be used. Biological samples may comprise lipoprotein such as HDL, LDL, VLDL, or CM, creatinine, uric acid, glucose, glutamate oxaloacetate transaminase (GOT) (aspartate aminotransferase (AST)), glutamate pyruvate transaminase (GPT) (alanine aminotransferase (ALT)), γ-glutamyl transpeptidase (γ-GTP), lactate dehydrogenase (LDH), alkaline phosphatase (ALP), creatine phosphokinase (CPK), or amylase (AMY). Examples thereof include, but are not limited to, body fluids, such as blood, blood serum, or blood plasma, and a diluted product thereof.

In the method of the present invention, the first target analyte and the second target analyte are subjected to the reaction as starting materials to generate reaction products each having the absorbance at a given wavelength, and the absorbance of such reaction product is measured. In this case, the first target analyte is converted into a reaction intermediate and then into the reaction product having the absorbance at a given wavelength through the reaction comprising at least two steps. The 2-step reaction advances with the aid of different reagent compositions. The second target analyte may be converted into the reaction product having the absorbance at a given wavelength through a 1-step reaction that does not involve formation of a reaction intermediate or through a 2-step reaction that involves formation of a reaction intermediate. The method of the present invention involves the use of two types of reagents and involves two steps. The term "reaction intermediate" used herein may refer to a substance that is converted from a target analyte or a substance resulting from the reaction of a target analyte and a reagent composition. In the present invention, the term "reagent" refers to a substance that comprises a reagent composition. The term "reagent composition" refers to, for example, a substance such as a surfactant or enzyme that constitutes a reagent. In the first step, a reaction intermediate is generated from the first target analyte with the aid of the first reagent. In the second step, subsequently, the reaction product originating from the first target analyte having the absorbance at a given wavelength is generated from a reaction intermediate originating from the first target analyte with the aid of the second reagent, and the reaction product originating from the second target analyte having the absorbance at a given wavelength is generated from the second target analyte. When the reaction whereby generating the reaction product originating from the second target analyte from the second target analyte does not involve formation of a reaction intermediate, in the second step, the reaction whereby generating the reaction product originating from the first target analyte from the reaction intermediate originating from the first target analyte advances substantially simultaneously with the reaction whereby generating the reaction product originating from the second target analyte from the second target analyte, and the reaction product originating from the first target analyte and the reaction product originating from the second target analyte are substantially simultaneously generated. In such a case, the absorption wavelength of the reaction product originating from the first target analyte needs to differ from that of the reaction product originating from the second target analyte, in order to simultaneously and differentially quantifying the first target analyte and the second target analyte. Specifically, the first measurement (measurement 1) and the second measurement (measurement 2) are carried out at different wavelengths in the second step, and both measurement 1 and measurement 2 are carried out at the end of the second step. When the reaction whereby generating the reaction product originating from the second target analyte from the second target analyte involves formation of a reaction intermediate, however, the reaction whereby generating the reaction product originating from the first target analyte from the reaction intermediate originating from the first target analyte in the second step advances faster than the reaction whereby generating the reaction product originating from the second target analyte from the second target analyte, and the reaction product originating from the first target analyte is generated prior to the generation of the reaction product originating from the second target analyte. In such a case, the absorption wavelength of the reaction product originating from the first target analyte may be the same or different from that of the reaction product originating from the second target analyte, and the first measurement (measurement 1) is carried out prior to the second measurement (measurement 2) in the second step. In the method of the present invention, preferably, quantification is carried out under parameters where the first measurement (measurement 1) is carried out prior to the second measurement (measurement 2).

In the method of the present invention, for example, a sequence of reactions consisting of the first target analyte → (reaction A) → the first reaction intermediate → (reaction B) → the first reaction product and the second target analyte → (reaction C) → the second reaction intermediate → (reaction D) → the second reaction product is employed. An arrow indicates a chemical reaction, and a reaction is, for example, an oxidation-reduction reaction utilizing an enzyme. In the method of the present invention, for example, 4 types of chemical reactions, i.e., reactions A, B, C, and D, take place. Reaction A and reaction C may be the same, and reaction B and reaction D may be the same. The same reaction refers to a reaction that takes place via the same chemical principle with the aid of the same reagent. Reactions A, B, C, and D may each comprise a plurality of reaction steps. In such a case, other reaction intermediates can further be generated in the aforementioned arrow-indicated reaction steps comprising the first target analyte → the first reaction intermediate → the first reaction product and the second target analyte → the second reaction intermediate → the second reaction product. In the method of the present invention, the first reaction intermediate is generated, but the second target analyte does not cause the reaction in the first step of the two steps, the first reaction product is generated from the first reaction intermediate, and the second target analyte is converted into the second reaction intermediate and then into the second reaction product in the second step. In this case, the rate of generating the second reaction product from the second target analyte is required to be slower than the rate of generating the first reaction product from the first reaction intermediate. Typically, the number of reaction steps for generating the second reaction product from the second target analyte is preferably greater than that for generating the first reaction product from the first reaction intermediate. The reaction rate for generating a different substance from a given substance varies depending on the type of reaction, and the reaction rate can be regulated by modifying the reaction parameters. In the two reactions such as the first reaction intermediate → the first reaction product and the second target analyte → the second reaction product, however, when the reaction such as the second target analyte → the second reaction product does not involve formation of a reaction intermediate, it is not always easy to maintain the reaction rate for the second target analyte → the second reaction product slower than the reaction rate for the first reaction intermediate → the first reaction product. In such a case, the reaction such as the second target analyte → the second reaction product may be advanced by a 2-step reaction involving formation of the second reaction intermediate, so that the reaction rate for the second target analyte → the second reaction product can be easily maintained slower than the reaction rate for the first reaction intermediate → the first reaction product. For example, concentrations or the like of reagents compositions for each reaction may be adjusted so as to maintain the rates of all chemical reactions at substantially the same levels, and the rate of the entire reactions simply depends on the number of steps throughout the reaction. Accordingly, reaction intermediates other than the first reaction intermediate and the second reaction intermediate may or may not be present, provided that the above requirement is fulfilled. The first reaction intermediate and the second reaction intermediate may be the same substances, and the first reaction product and the second reaction product may be the same substances. The method of the present invention involves the use of two types of reagents and involves two steps. A step refers to a step wherein a series of reactions advances with the addition of a single type of reagent. The first step refers to a step from the addition of the first reagent up to the addition of the second reagent. The second step refers to a step from the addition of the second reagent up to the completion of the quantification. In the method of the present invention, the first target analyte reacts with the reagent composition contained in the first reagent to generate the first reaction intermediate in the first step. Subsequently, in the second step, the first reaction intermediate reacts with the reagent composition contained in the second reagent to generate the first reaction product, and the second target analyte reacts with the reagent composition contained in the second reagent to generate the second reaction intermediate and then the second reaction product. When the reaction comprising the first target analyte → the first reaction intermediate and the reaction comprising the second target analyte → the second reaction intermediate are advanced based on the same principle, the first reagents comprises at least a reagent composition used for the reaction of interest.

In the first step, the reaction, the first target analyte → (reaction A) → the first reaction intermediate, solely takes place. In the second step, the reaction comprising the first reaction intermediate → (reaction B) → the first reaction product, the reaction comprising the second target analyte → (reaction C) → the second reaction intermediate, and the reaction comprising the second reaction intermediate → (reaction D) → the second reaction product take place. In the second step, the first reaction product and the second reaction product are measured. The measurement can be optically carried out using light of the absorption wavelength specific to the first reaction product and to the second reaction product. In this case, if the absorption wavelength of the first reaction product is the same as that of the second reaction product, the first reaction product and the second reaction product can be measured using the light of the same wavelength.

When the second reagent is added in the second step, the reaction, the first reaction intermediate → (reaction B) → the first reaction product, advances at first, and the first measurement (measurement 1) is then carried out. Via measurement 1, the first reaction product originating from the first target analyte is measured, and the first target analyte can be quantified. In the second step, following the reaction, the first reaction intermediate → (reaction B) → the first reaction product, the reaction, the second reaction intermediate → (reaction D) → the second reaction product, takes place. At the last stage of the second step, the second measurement (measurement 2) is carried out. The measurement 2 enables quantification of the second target analyte. When the absorption wavelengths are the same as in the case where the first reaction product and the second reaction product are the same substances, the measured value obtained via measurement 1 is added to the measure value obtained via measurement 2. This results in the measurement of both the first reaction product originating from the first target analyte and the second reaction product originating from the second target analyte in the second measurement. Thus, the measured value obtained via measurement 1 may be subtracted from the measured value obtained via measurement 2 to quantify the second target analyte. This enables simultaneous and differential quantification of the first target analyte and the second target analyte in the second step.

Simultaneous quantification of two substances refers to a procedure of obtaining measured values of two substances via a single assay operation. The term "a single assay operation" used herein refers to a series of continuing process from assay of biological samples up to the completion of assay when the necessary number of measured values is obtained. A single assay operation comprises the addition of reagents several times and the acquisition of measured values; however, separation processes via centrifugation or formation of complexes are excluded. Preferably, a single assay operation is completed in a sole assay tube or well. For example, two measurements, i.e., measurement 1 and measurement 2, are carried out in the second step, as shown in Fig. 1 that represents the principle of the present invention. In the present invention, such two measurements are referred to as "a single assay operation." A single assay operation consisting of measurement 1 and measurement 2 enables simultaneous quantification of two target analytes.

Hereafter, the method of the present invention is concretely described with reference to a case where target analytes are mainly lipid components in the lipoprotein, such as cholesterol or triglyceride components. Those skilled in the art would be capable of adequately determining reagents, wavelength for the measurement, and other conditions employed for assaying other analytes, based on the following description concerning the assay of lipid components.

The present invention relates to a method for simultaneously assaying two different substances in a biological sample comprising quantifying two different substances in a given lipid component in a biological sample, which is the analyte sample, using the absorbance of a pigment resulting from lipoprotein processing as an indicator via a single assay operation. Further, the present invention relates to a method that inhibits a reagent from spontaneously developing color due to air oxidation, improves the stability of a reagent or reagent composition, and obtains stable results even if a reagent is allowed to stand for a long period of time. According to the method of the present invention, lipoprotein in a biological sample is processed to generate hydrogen peroxide, the resulting hydrogen peroxide is converted into a quinone pigment, and the absorbance of the quinone pigment is then measured to assay the concentration of components in the lipoprotein in the biological sample. The method of the present invention comprises the first step of processing the given lipoprotein, which is a target analyte in a biological sample (i.e., the first target analyte) to generate hydrogen peroxide and the second step of converting hydrogen peroxide obtained in the first step (i.e., the first reaction intermediate) into a quinone pigment (i.e., the first reaction product), processing the other target analyte, lipoprotein (i.e., the second target analyte), to generate hydrogen peroxide (i.e., the second reaction intermediate), and converting the resulting hydrogen peroxide into a quinone pigment (i.e., the second reaction product). According to the method of the present invention, specifically, two types of target analytes are independently processed in separate steps, hydrogen peroxides resulting from such processing are converted into quinone pigments, and the resulting quinone pigments are detected. A series of such processing is carried out in a single step. In such a case, the first reaction intermediate and the second reaction intermediate are the same substances, and the first reaction product and the second reaction product are the same substances.

The term "lipid component" refers to cholesterol or triglyceride that constitutes a lipoprotein.

When target analytes are given twolipid components consisting of two types of lipoproteins, hydrogen peroxide is generated by the first target-analyte-based reaction in the first step, the hydrogen peroxide generated in the first step changes the absorbance of the reaction solution in the second step, the second target-analyte-based reaction takes place, and changes in the absorbance of the reaction solution due to such reaction are then measured. The total extent of change in the absorbance in the second step is equivalent to the sum of the amount of the first target analyte and those of the second target analyte, and the extent of change in the absorbance relative to the amount of hydrogen peroxide generated in the second step is equivalent to the amount of the second target analyte. The parameters for analyzing the changes in the absorbance using an automated analyzer may be changed to simultaneously obtain two measured values via a single assay operation. The assay value of the first target analyte can be obtained by subtracting a measured value for a target analyte determined based on the extent of change in the absorbance at two time points after the addition of the second reagent from the measured value determined based on the total extent of change in the absorbance in the second step. When target analytes are one lipid component and the total amount of lipid components in the given lipoprotein, hydrogen peroxide is generated by the reaction based on lipoproteins other than the given lipoprotein in the first step, the hydrogen peroxide generated in the first step changes the absorbance of the reaction solution in the second step, the reaction based on the given lipoprotein takes place, and changes in the absorbance of the reaction solution due to such reaction are then measured. The extent of change in the absorbance throughout the second step is equivalent to the total amount of lipid components, and the extent of change in the absorbance relative to the amount of hydrogen peroxide generated in the second step is equivalent to the amount of the given lipoprotein.

In the present invention, processing of lipoprotein refers to processing of lipoprotein with a surfactant, other additive, or enzyme. When lipoprotein is processed with a surfactant, a lipid component in the lipoprotein is released. Upon further processing with an enzyme, hydrogen peroxide is generated. Specifically, processing of lipoprotein comprises a series of processing from release of a lipid component from a lipoprotein to generation of hydrogen peroxide. Processing of a lipid component refers to processing of free cholesterol or triglyceride with an enzyme to generate hydrogen peroxide. The resulting hydrogen peroxide is then converted into a quinone pigment with the aid of peroxidase.

In the present invention, hydrogen peroxide is generated in the first step by the action of a reagent composition that reacts with a lipoprotein, which is the first target analyte in the biological sample, or a lipoprotein other than the given lipoprotein that undergoes the reaction in the second step (i.e., the second target analyte). Since the first reagent used in the first step does not comprise a set of reagent compositions associated with generation of a quinone pigment, the resulting hydrogen peroxide is not converted into a quinone pigment. In the second step, a lipid component is processed with a reagent composition that reacts with a lipoprotein, which at least becomes the second target analyte, and hydrogen peroxide is then generated by such reaction. When the second reagent used in the second step is added to the assay system, a set of reagent compositions associated with generation of a quinone pigment is contained in the assay system, simultaneously with processing lipoproteins as the second target analyte, hydrogen peroxide generated in the first step that is present in the assay system is converted into a quinone pigment. When the second step is initiated, the assay system contains hydrogen peroxide generated from a lipid component in the first target analyte generated in the first step or a lipoprotein other than the given lipoprotein that reacts in the second step, and the resulting hydrogen peroxide is converted into a quinone pigment simultaneously with the initiation of the second step. In contrast, hydrogen peroxide resulting from processing of the second target analyte in the second step is converted into a quinone pigment upon its generation. In the second step, the amount of hydrogen peroxide resulting from processing of the second target analyte increases along with a progress of processing of a lipid component with an enzyme with the elapse of time. Accordingly, hydrogen peroxide resulting from processing of the second target analyte in the second step is converted into a quinone pigment, and the amount of the quinone pigment accordingly increases with the elapse of time. Changes in the absorbance caused by a quinone pigment that occurred immediately after the initiation of the second step reflect the amount of hydrogen peroxide generated in the first step, i.e., the amount of a lipid component in the first target analyte or a lipoprotein other than the given lipoprotein that reacts in the second step. The total extent of change in the absorbance caused by a quinone pigment at the end of the second step reflect the amount of hydrogen peroxide generated in the second step, in addition to the amount of hydrogen peroxide generated in the first step, i.e., the amount of a lipid component in the second target analyte. In other words, the total extent of change in the absorbance in the second step are equivalent to the value, which reflects the sum of the amount of the first target analyte in the biological sample and the amount of a lipid component in the second target analyte or a value that reflects the total amount of lipid components, and changes in the absorbance relative to the amount of hydrogen peroxide generated in the second step is a value that reflects the amount of a lipid component in the second target analyte. Based on such two changes in the adsorption, i.e., the total extent of change in the absorbance in the second step and changes in the absorbance relative to the amount of hydrogen peroxide generated in the second step, the amounts of two different substances in a given lipid component of a biological sample can be simultaneously assayed. The assay value of the first target analyte can be obtained by subtracting a measured value for a target analyte determined based on the extent of change in the absorbance between two time points after the addition of the second reagent from the measured value determined based on the total extent of change in the absorbance in the second step. In the method of the present invention, different reagents are used in the first step and in the second step. By incorporating features in the compositions of the two reagents, mainly the formulation of a reagent composition associated with generation of a quinone pigment, reagents can be stabilized. This can also produce stable results in the method for assaying cholesterol. The term "reagent composition" refers to a composition of a reagent associated with a given chemical reaction and/or a reagent required for performing a chemical reaction of a buffer or the like. In the method of the present invention, a liquid reagent supplied in a liquid state can be stabilized. Hydrogen peroxide generated by the action of a reagent composition generates a colored quinone (i.e., a quinone pigment) in the presence of peroxidase, 4-aminoantipyrine, and a phenol or aniline hydrogen donor compound. When a reagent contains peroxidase, 4-aminoantipyrine, and a phenol or aniline hydrogen donor compound in a liquid state, a quinone pigment is generated with the elapse of time due to the influence of air oxidation even in the absence of hydrogen peroxide, and a reagent spontaneously develops color disadvantageously. Thus, a reagent or reagent composition for assaying a lipid component cannot be maintained stable, and stability of assay cannot be assured. In the present invention, all of peroxidase, 4-aminoantipyrine, and a phenol or aniline hydrogen donor compound should not be contained in one of the two reagents. Instead, all of peroxidase, 4-aminoantipyrine, and a phenol or aniline hydrogen donor compound are to be added to the system when two reagents are added to the assay system.

Cholesterols contained in the lipoprotein, which are the target analytes of the method according to the present invention, are classified into ester cholesterol (cholesterol ester) and free cholesterol. The term "cholesterol" used herein refers to both cholesterols.

The term "the value reflecting the amount of a lipid component" refers to a value that is obtained when the concentration or absolute amount of cholesterol or triglyceride in the lipoprotein in a biological sample is quantified. The method for assay is not limited. When a plurality of measurements are carried out and a value equivalent to the concentration or absolute amount of a lipid component in the lipoprotein in the biological sample, such as a proportional or inversely-proportional value, is attained in the end, such value is designated as "the value reflecting the amount of a lipid component." An example of such measured value is an absorbance resulting from a compound generated via a series of processing of cholesterol of the lipoprotein with a given agent. Such measured value refers to an absolute or variable amount.

For example, changes in the absorbance in the second step shown in Fig. 1 are equivalent to the sum of the absorbance resulting from conversion of hydrogen peroxide, which is generated by the processing in the first step, into a quinone pigment and the absorbance resulting from conversion of hydrogen peroxide, which is generated by the processing in the second step, into a quinone pigment. Based on the changes in the absorbance obtained by the difference between the absorbance attained in measurement 2 and that attained in measurement 1 in the second step shown in Fig. 1, the absorbance reflecting the amount of a lipid component in the second target analyte can be attained. The total absorbance attained in measurement 2 of the second step is the sum of the absorbance equivalent to the amount of a lipid component in the second target analyte added to that in the first target analyte or the absorbance equivalent to the total amount of the lipid component. In fact, an accurate measured value is determined based on a value attained by subtracting the absorbance before the addition of the second reagent from the absorbance attained in measurement 2.

When one of the two types of meaured values attained via a single assay operation is the total amount of a given lipid component (i.e., total cholesterol or total triglyceride) in a biological sample, the total absorbance attained via measurement 2 in the second step reflects the amount of such lipid component.

Processing in the first step is carried out by degrading a lipid component via an enzyme reaction in the presence of a surfactant that acts on the given lipoprotein. Hydrogen peroxide resulting therefrom is maintained until the second step without being eliminated or detected. The fact that "a surfactant that acts on..." refers to the phenomenon such that a surfactant degrades a lipoprotein and releases cholesterol in the lipoprotein.
(1) Simultaneous assay of LDL cholesterol and total cholesterol in a cholesterol assay system
   In the first step, a lipid component is processed with a surfactant that reacts with lipoproteins other than LDL in a biological sample, cholesterol esterase, and cholesterol oxidase to generate hydrogen peroxide. The term "lipoproteins other than LDL" refers to, for example, HDL, VLDL, or CM.
   In the second step, a lipid component is processed with a surfactant and an enzyme that reacts at least with LDL, and hydrogen peroxide is then generated resulting therefrom. The total extent of change in the absorbance in the second step reflects the amount of total cholesterol in a biological sample, and changes in the absorbance relative to the amount of hydrogen peroxide generated in the second step reflect the amount of LDL cholesterol. Based on the changes in the above two absorbance values, i.e., the total extent of change in the absorbance in the second step and changes in the absorbance relative to the amount of hydrogen peroxide generated in the second step, the amounts of LDL cholesterol and of total cholesterol in a biological sample can be simultaneously assayed.
   An example of a specific method for allowing cholesterols contained in lipoproteins other than LDL, such as HDL, VLDL, or CM lipoproteins, to selectively react is described below.
   In the presence of a surfactant that acts on lipoproteins other than LDL, specifically, cholesterol esterase and cholesterol oxidase are allowed to react in order to generate hydrogen peroxide.
   The cholesterol esterase concentration in the reaction solution used in the first step is preferably about 0.2 to 2.0 IU/ml, and those produced by *Pseudomonas* bacteria are effective. Also, the cholesterol oxidase concentration is preferably about 0.1 to 0.7 IU/ml, and use of those derived from bacteria or yeast is preferable.
   A preferable example of a surfactant that acts on lipoproteins other than LDL used in the first step is a surfactant having an HLB value of 13 to 14, and preferably a polyalkylene oxide derivative having an HLB value of 13 to 14. Examples of derivatives include a condensation product of a higher alcohol, a condensation product of higher fatty acid, a condensation product of higher fatty acid amide, a condensation product of higher alkyl amine, a condensation product of higher alkyl mercaptan, and a condensation product of alkylphenol. A method for determining an HLB value of a surfactant is well-known, and it is described in, for example, "Shin kaimen kasseizai (New Surfactant)," Hiroshi Horiuchi, 1986, Sankyo Publishing Co., Ltd.
   Specific examples of preferable polyalkylene oxide derivatives having an HLB value of 13 to 14 include, but are not limited to, compounds, such as polyoxyethylene lauryl ether, polyoxyethylene cetyl ether, polyoxyethylene oleyl ether, polyoxyethylene higher alcohol ether, polyoxyethylene octylphenyl ether, polyoxyethylene nonylphenyl ether, and polyoxyethylenebenzyl phenyl ether.
   An example of a surfactant used in the first step is a polyoxyethylene derivative, and more specifically Emulgen B-66 (Kao Corporation) having an HLB value of 13.2.
   The concentration of the surfactant used in the first step is preferably about 0.1 to 10 g/l, and more preferably about 0.5 to 5.0 g/l.
   In the first step, a bile acid derivative and/or an amphoteric surfactant can be used as a reagent composition that acts on lipoproteins other than LDL in the presence of a polymer of 1-olefin and maleic acid, acrylic acid, or methacrylic acid having 6 to 32 carbon atoms or a polymer compound comprising acid amide or ester thereof. In this case, the molecular weight of such polymer compound is preferably 5,000 to 500,000 daltons, and the concentration of the polymer compound is preferably 0.001% to 1%. The concentration of the bile acid derivative and of an amphoteric surfactant are determined in accordance with a type of a selected surfactant.
   The first step is preferably carried out in a buffer having a pH of 5 to 9, and an amine-containing buffer, such as a Tris, triethanolamine, or Good's buffer, is preferable. Good's buffers, such as Bis-Tris, PIPES, MOPSO, BES, HEPES, and POPSO, are particularly preferable, and the concentration of the buffer is preferably about 10 to 500 mM.
   The reaction temperature in the first step is preferably about 30°C to 40°C, with 37°C being the most preferable. The reaction time (i.e., the duration from the addition of the first reagent to the addition of the second reagent) may be about 2 to 10 minutes, with 5 minutes being preferable.
   According to the method of the present invention, the first step is preferably carried out in the presence of albumin. Albumin is not particularly limited, and commercially available albumin such as serum albumin can be preferably used, with fatty acid-free albumin being particularly preferable. An albumin origin is not particularly limited, it may originate from any animal such as a human, cow, pig, or horse, and use of common bovine serum albumin is particularly preferable. The concentration of the albumin in the reaction solution used in the first step is preferably 0.1 to 5.0 g/dl, and more preferably 0.3 to 3.0 g/d1.
   Thus, the first reagent used in the first step at least comprises a surfactant, cholesterol esterase, and cholesterol oxidase. Such reagent may further comprise an adequate buffer, albumin, and/or a polymer compound. The reagent used in the first step does not comprise all reagent compositions associated with generation of a quinone pigment. The reagent comprises either 4-aminoantipyrine, or a phenol- or aniline-hydrogen donor compound. Also, the first reagent used in the first step does not comprise peroxidase.
   In the first step, hydrogen peroxide is generated in accordance with the amount of cholesterol in lipoproteins other than LDL in a biological sample, and such hydrogen peroxide is carried over to the second step without being eliminated or detected.
   In the subsequent second step, hydrogen peroxide generated from cholesterol in lipoproteins other than LDL processed in the first step is quantified, and cholesterol in LDL that remained at the end of the first step is processed and then quantified.
   Cholesterol in LDL is processed by processing LDL with a surfactant that acts at least on LDL. Cholesterol in LDL generates hydrogen peroxide by the action of such surfactant, cholesterol esterase, and cholesterol oxidase. Cholesterol esterase and cholesterol oxidase are contained in the first reagent used in the first step, and those that have been added to the assay system in the first step may be used. The second reagent used in the second step may comprise cholesterol esterase and cholesterol oxidase. A surfactant that acts at least on LDL is preferably a surfactant that acts selectively on LDL. A surfactant that acts on any lipoprotein may also be used.
   The assayed value of LDL is determined based on changes in the absorbance after the addition of the second reagent. Accordingly, the reaction rate, i.e., the reaction intensity of a surfactant used, would affect the accuracy of the assayed value. Thus, a surfactant having adequate reaction intensity is preferably selected.
   A preferable example of a surfactant that acts selectively on LDL or acts on any lipoproteins is a polyalkylene oxide derivative, which is not used in the first reagent. Examples of derivatives include a condensation product of a higher alcohol, a condensation product of higher fatty acid, a condensation product of higher fatty acid amide, a condensation product of higher alkyl amine, a condensation product of higher alkyl mercaptan, and a condensation product of alkylphenol.
   Specific examples of preferable polyalkylene oxide derivatives include compounds that are not used in the first reagent, such as polyoxyethylene lauryl ether, polyoxyethylene cetyl ether, polyoxyethylene oleyl ether, polyoxyethylene higher alcohol ether, polyoxyethylene octylphenyl ether, polyoxyethylene nonylphenyl ether, and polyoxyethylenebenzyl phenyl ether. An example of a polyalkylene oxide derivative used in the second step is polyoxyethylene lauryl alcohol, specifically, Polidocanol (Thesit) having an HLB value of 13.3 (Roche Diagnostics). Also, a copolymer of polyoxyethylene-polyoxypropylene can further be added to the polyalkylene oxide derivative. A copolymer of polyoxyethylene-polyoxypropylene may be a random copolymer or block polymer of polyoxyethylene and polyoxypropylene. The molecular weight of the copolymer of polyoxyethylene-polyoxypropylene is preferably 2,750 daltons or higher. The HLB value of the copolymer of polyoxyethylene-polyoxypropylene is preferably 1 to 6. Examples of a copolymer of polyoxyethylene-polyoxypropylene used in the second step include Pluronic L-121, Pluronic L-122, Pluronic L-101, Pluronic P-103, and Pluronic F-108 (Asahi Denka Co., Ltd.).
   The concentration of the surfactant used in the second step is preferably about 0.1 to 100 g/l, and more preferably about 1 to 50 g/l.
   Other preferable reaction parameters in the second step are the same as those preferable in the first step. In the second step of the method according to the present invention, hydrogen peroxide derived from cholesterol in lipoproteins other than LDL contained in the assay system immediately after the initiation of the second step is converted into a quinone pigment, and hydrogen peroxide is generated from cholesterol in LDL along with the advancement of the second step to convert such hydrogen peroxide into a quinone pigment. An elevated absorbance value caused by a quinone pigment generated via processing of lipoproteins other than LDL begins simultaneously with the addition of the second reagent, and it rapidly advances and is completed within a short period of time. In contrast, LDL is processed after the addition of the second reagent, hydrogen peroxide is generated, and a quinone pigment is then generated. Accordingly, the absorbance that reflects the amount of LDL begins to elevate after some time has passed after the addition of the second reagent, and the rate of such elevation is not large. That is, an elevated absorbance in the second step is a 2-phase elevation of a rapid elevation immediately after the initiation of the second step and a mild elevation. The first rapid elevation reflects the amount of lipoproteins other than LDL, and the latter mild elevation reflects the amount of LDL. In the reaction from the initiation to the completion of the second step, the time is represented by a horizontal axis, the amount of quinone pigment generated is represented by a horizontal axis, and the average rate of the entire quinone pigment generation is designated as "t." An increase of a quinone pigment resulting from a reaction that is carried out at a faster rate than "t" is referred to as "a rapid increase," and an increase of a quinone pigment resulting from a reaction that is carried out at a slower rate than "t" is referred to as "a mild increase." "A rapid increase" takes place in a short period of time, and "a mild increase" takes place over a relatively long time. In Fig. 1, a rapid elevation in the absorbance immediately after the addition of the second reagent refers to "a rapid increase," and the elevation of the absorbance from measurement 1 to measurement 2 refers to "a mild increase." "A rapid increase" takes place within 0 to 60 seconds, and preferably within 30 seconds, after the addition of the second reagent.
   Accordingly, it is preferable that a quinone pigment originating from cholesterol in lipoproteins other than LDL be measured separately from a quinone pigment originating from cholesterol in LDL and the generation of a quinone pigment originating from cholesterol in LDL be completed within 30 seconds to 5 minutes after the addition of the second reagent, so that the amount of cholesterol in LDL can be accurately quantified.
   The amount of cholesterol in lipoproteins other than LDL can be determined by quantifying hydrogen peroxide generated by the actions of cholesterol esterase and of cholesterol oxidase in the first step. Also, the amount of cholesterol in LDL can be determined by adding a surfactant that acts on LDL at least in the second step and quantifying hydrogen peroxide generated by the actions of such surfactant and cholesterol esterase and of cholesterol oxidase added in the first step. Hydrogen peroxide can be quantified by converting the generated hydrogen peroxide into colored quinone by an oxidation condensation reaction of 4-aminoantipyrine and a phenol or aniline hydrogen donor compound with the aid of peroxidase, and conducting a measurement at 400 to 700 nm. When the second reagent used in the second step is added to the assay system, all the reagent compositions associated with generation of a quinone pigment of peroxidase, 4-aminoantipyrine, and a phenol or aniline hydrogen donor compound are included in the system. Therefore, the second reagent used in the second step comprises at least a surfactant that acts on LDL and a reagent composition that is not contained in the first reagent used in the first step, among peroxidase, 4-aminoantipyrine, and a hydrogen donor compound (phenol or aniline). Further, the second reagent used in the second step may comprise any of a buffer, albumin, cholesterol esterase, or cholesterol oxidase.
   The measured absorbance of the colored quinone generated in the reaction of the second step is a sum of the absorbance originating from hydrogen peroxide in the reaction of the first step and the absorbance originating from hydrogen peroxide in the reaction of the second step. Such value indicates the amounts of cholesterol in all lipoproteins in a biological sample. Also, a value attained by subtracting the absorbance of hydrogen peroxide generated in the first step from the total amount of the absorbance, i.e., the quantified amount of hydrogen peroxide generated in the second step, indicates the amount of cholesterol in LDL.
   Among hydrogen donor compounds, examples of aniline hydrogen donor compounds include N-(2-hydroxy-3-sulfopropyl)-3,5-dimethoxyaniline (HDAOS), N-ethyl-N-sulfopropyl-3-methoxyaniline (ADPS), N-ethyl-N-sulfopropylaniline (ALPS), N-ethyl-N-sulfopropyl-3,5-dimethoxyaniline (DAPS), N-sulfopropyl-3,5-dimethoxyaniline (HDAPS), N-ethyl-N-sulfopropyl-3,5-dimethylaniline (MAPS), N-ethyl-N-sulfopropyl-3-methylaniline (TOPS), N-ethyl-N-(2-hydroxy-3-sulfopropyl)-3-methoxyaniline (ADOS), N-ethyl-N-(2-hydroxy-3-sulfopropyl)aniline (ALOS), N-ethyl-N-(2-hydroxy-3-sulfopropyl)-3,5-dimethoxyaniline (DAOS), N-ethyl-N-(2-hydroxy-3-sulfopropyl)-3,5-dimethylaniline (MAOS), N-ethyl-N-(2-hydroxy-3-sulfopropyl)-3-methylaniline (TOOS), and N-sulfopropylaniline (HALPS).
   When hydrogen peroxide is converted into a quinone pigment in the reaction solution in the second step, the concentration of peroxidase is preferably 0.1 to 3.0 IU/ml, that of 4-aminoantipyrine is preferably 0.3 to 3.0 mmol/l, and that of a phenol or aniline hydrogen donor compound is preferably 0.5 to 2.0 mmol/l.
   When the second reagent is added in the second step, all reagent compositions associated with generation of a quinone pigment are included in the system. Thus, hydrogen peroxide generated in the first step is converted into a quinone pigment at an early stage of the second step. Simultaneously, cholesterol in LDL is treated with a surfactant that acts on LDL contained in the second reagent and cholesterol esterase and cholesterol oxidase contained in the assay system to generate hydrogen peroxide. Hydrogen peroxide originating from cholesterol in LDL is converted into a quinone pigment by the action of a reagent composition associated with generation of a quinone pigment contained in the assay system upon its generation. Thus, the amount of a quinone pigment increases with the elapse of time. As shown in Fig. 1, accordingly, in the second step, the absorbance rapidly increases simultaneously with the initiation of the second step, and a mild increase then continues with the elapse of time. The rapidly increased absorbance is measured via the first measurement, and the mildly increased absorbance with the elapse of time is measure via the second measurement. The measured value attained via the second measurement reflects the amount of total cholesterol, and the difference between the second measurement and the first measurement indicates the amount of cholesterol in LDL.
(2) Simultaneous assay of HDL cholesterol and total cholesterol in cholesterol assay system
   Assay can be carried out with the use of a reagent composition that reacts with lipoproteins other than HDL in the first step in (1) above and a reagent composition that at least reacts with HDL in the second step. In the first step, lipoproteins are processed with cholesterol esterase and cholesterol oxidase to generate hydrogen peroxide in the absence of a surfactant that reacts specifically with HDL in a biological sample. In the second step, lipoproteins are processed with a surfactant that reacts at least with HDL and an enzyme in a biological sample, and hydrogen peroxide is then generated. The total extent of change in the absorbance in the second step reflects the amount of total cholesterol in a biological sample, and the extent of change in the absorbance relative to the amount of hydrogen peroxide generated in the second step reflects the amount of cholesterol in HDL. Based on the changes in the above two absorbance values, i.e., the total extent of change in the absorbance in the second step and changes in the absorbance relative to the amount of hydrogen peroxide generated in the second step, the amounts of HDL cholesterol and of total cholesterol in a biological sample can be simultaneously assayed.
   Specifically, in the first step, lipoproteins are processed with cholesterol esterase and cholesterol oxidase to generate hydrogen peroxide in the absence of a surfactant that reacts specifically with HDL. Also, a surfactant that reacts with lipoproteins other than HDL in a biological sample can be used in the first step, in addition to such cholesterol esterase and cholesterol oxidase. Preferable examples of a surfactant that acts on lipoproteins other than HDL used in the first step include a copolymer of polyoxyethylene-polyoxypropylene and a polyalkylene oxide derivative. The molecular weight of a copolymer of polyoxyethylene-polyoxypropylene is preferably 5,000 daltons or higher, and that of a hydrophobic group is preferably 3,000 daltons or lower. In order to allow lipoproteins other than HDL to coagulate, a coagulant and/or a divalent metal salt may be added. Examples of a coagulant include heparin, phosphotungstic acid, dextran sulfate, sulfated cyclodextrin, sulfated oligosaccharide, a salt of any thereof, and polyethylene glycol. Heparin and salt thereof having the concentration of 0.02 to 10 mM, phosphotungstic acid and salt thereof having the concentration of 0.1 to 10 mM and the molecular weight 4,000 to 8,000 daltons, dextran sulfate and salt thereof having the concentration of 0.01 to 5 mM and the molecular weight of 10,000 to 500,000 daltons, and polyethylene glycol having the concentration of 0.3 to 100 mM and the molecular weight of 4,000 to 25,000 are preferable. In order to promote the elimination of lipoproteins other than HDL in the first step, a divalent metal ion can further be added as a reagent composition. As a divalent metal ion, a copper, iron, or magnesium ion is preferable, with a magnesium ion being particularly preferable. The concentration of a divalent metal ion is preferably 5 to 200 mmol/l. The concentration of cholesterol esterase in the reaction solution of the first step is preferably about 0.2 to 2.0 IU/ml, and use of those produced by *Pseudomonas* bacteria is effective. The concentration of cholesterol oxidase is preferably about 0.1 to 0.7 IU/ml, and use of cholesterol oxidase having the molecular weight of 60 kilodaltons or lower is preferable.
   In the first step, hydrogen peroxide is generated in accordance with the amount of cholesterol in lipoproteins other than HDL in a biological sample, and such hydrogen peroxide is carried over to the second step without being eliminated or detected.
   The reagent used in the first step does not comprise all reagent compositions associated with generation of a quinone pigment. The reagent comprises either 4-aminoantipyrine or a phenol or aniline hydrogen donor compound. Also, the first reagent used in the first step does not comprise peroxidase.
   In the subsequent second step, hydrogen peroxide generated from cholesterol in lipoproteins other than HDL processed in the first step is quantified, and cholesterol in HDL that remained at the end of the first step is processed and then quantified.
   Cholesterol in HDL is processed by processing HDL with a surfactant that acts on at least HDL. Cholesterol in HDL generates hydrogen peroxide by the action of such surfactant, cholesterol esterase, and cholesterol oxidase. Cholesterol esterase and cholesterol oxidase are contained in the first reagent used in the first step, and those that have been added to the assay system in the first step may be used. The second reagent used in the second step may comprise cholesterol esterase and cholesterol oxidase. A surfactant that acts at least on HDL is preferably a surfactant that acts selectively on HDL. A surfactant that acts on any lipoprotein may also be used.
   As the surfactants, a nonionic surfactant having an HLB value of 13 to 14 is preferable, and a polyalkylene oxide derivative is particularly preferable. Examples of a derivative include a condensation product of a higher alcohol, a higher fatty acid complex, a condensation product of higher fatty acid amide, a condensation product of higher alkyl amine, a condensation product of higher alkyl mercaptan, and a condensation product of alkylphenol.
   Specific examples of preferable polyalkylene oxide derivatives having an HLB value of 13 to 14 include, but are not limited to, compounds, such as polyoxyethylene lauryl ether, polyoxyethylene cetyl ether, polyoxyethylene oleyl ether, polyoxyethylene higher alcohol ether, polyoxyethylene octylphenyl ether, polyoxyethylene nonylphenyl ether, and polyoxyethylenebenzyl phenyl ether. Also, a plurality of surfactants may be mixed in order to adjust an HLB value within such range.
   When the second reagent used in the second step is added to the assay system, all the reagent compositions associated with generation of a quinone pigment of peroxidase, 4-aminoantipyrine, and a phenol or aniline hydrogen donor compound are included in the system. Specifically, the second reagent used in the second step comprises at least a surfactant that acts on HDL and a reagent composition that is not contained in the first reagent used in the first step, among peroxidase, 4-aminoantipyrine, and a hydrogen donor compound (phenol or aniline).
   As shown in Fig. 1, accordingly, in the second step, the absorbance rapidly increases simultaneously with the initiation of the second step, and a mild increase then continues with the elapse of time. The rapidly increased absorbance is measured via the first measurement, and the mildly increased absorbance with the elapse of time is measured via the second measurement. The measured value attained via the second measurement reflects the amount of total cholesterol, and the difference between the second measurement and the first measurement indicates the amount of cholesterol in HDL.
(3) Simultaneous assay of HDL cholesterol and LDL cholesterol in cholesterol assay system
   Assay can be carried out with the use of a reagent composition that reacts with HDL in the first step and a reagent composition that reacts with LDL in the second step in (1) above.
   In the first step, lipoproteins are processed with cholesterol esterase and cholesterol oxidase to generate hydrogen peroxide in the presence of a surfactant that reacts specifically with HDL or a reagent composition that allows lipoproteins other than HDL to coagulate in a biological sample. The reagent used in the first step does not comprise all reagent compositions associated with generation of a quinone pigment. The reagent comprises either 4-aminoantipyrine or a phenol or aniline hydrogen donor compound. Also, the first reagent used in the first step does not comprise peroxidase. In the second step, lipoproteins are processed with a surfactant and an enzyme that react with LDL, and hydrogen peroxide is then generated. The total extent of change in the absorbance in the second step reflects the sum of the amounts of HDL cholesterol and LDL cholesterol in a biological sample, and the extent of change in the absorbance relative to the amount of hydrogen peroxide generated in the second step reflects the amount of cholesterol in LDL. The amount based on the changes in the absorbance attained in the second step may be subtracted from the amount based on the total extent of change in the absorbance to determine the amount of HDL cholesterol.
   A nonionic surfactant having an HLB value of 13 to 14 is preferable as a reagent composition that acts on HDL, and a polyalkylene oxide derivative is particularly preferable. Examples of a derivative include a condensation product of a higher alcohol, a higher fatty acid complex, a condensation product of higher fatty acid amide, a condensation product of higher alkyl amine, a condensation product of higher alkyl mercaptan, and a condensation product of alkylphenol.
   Specific examples of preferable polyalkylene oxide derivatives having an HLB value of 13 to 14 include, but are not limited to, compounds, such as polyoxyethylene lauryl ether, polyoxyethylene cetyl ether, polyoxyethylene oleyl ether, polyoxyethylene higher alcohol ether, polyoxyethylene octylphenyl ether, polyoxyethylene nonylphenyl ether, and polyoxyethylenebenzyl phenyl ether. Also, a plurality of surfactants may be mixed in order to adjust the HLB value within such range. As a substance that acts on the HDL, a reagent that allows lipoproteins other than HDL to coagulate, an antibody against lipoproteins other than HDL, and the like can be used. As a reagent composition that allows lipoproteins other than HDL to coagulate, a substance comprising a lipoprotein coagulant and/or a divalent cation may be used. Examples of a coagulant include heparin, phosphotungstic acid, dextran sulfate, sulfated cyclodextrin, sulfated oligosaccharide, or salt of any thereof, and polyethylene glycol. Heparin and salt thereof having the concentration of 0.02 to 10 mM, phosphotungstic acid and salt thereof having the concentration of 0.1 to 10 mM and the molecular weight 4,000 to 8,000 daltons, dextran sulfate and salt thereof having the concentration of 0.01 to 5 mM and the molecular weight of 10,000 to 500,000 daltons, and polyethylene glycol having the concentration of 0.3 to 100 mM and the molecular weight of 4,000 to 25,000 are preferable. Such coagulant may be used alone or in combinations of two or more. Further, a coagulant may be used in combination with a surfactant having an HLB value of 13 to 14.
   Examples of a divalent cation that can be used include magnesium, manganese, nickel, calcium, and salt thereof. The concentration is preferably 0.1 to 50 mM.
   In the second step, a reagent composition that reacts selectively with LDL is used. Specific examples thereof include a copolymer of polyoxyethylene-polyoxypropylene and a polyalkylene oxide derivative. Specific conditions are as described in (1) above.
   When the second reagent used in the second step is added to the assay system, all reagent compositions associated with generation of a quinone pigment of peroxidase, 4-aminoantipyrine, and a phenol or aniline hydrogen donor compound are contained in the system. Specifically, the second reagent used in the second step comprises at least a surfactant that acts on LDL and a reagent composition that is not contained in the first reagent used in the first step, among peroxidase, 4-aminoantipyrine, and a hydrogen donor compound (phenol or aniline).
   As shown in Fig. 1, in the second step, the absorbance rapidly increases simultaneously with the initiation of the second step, and a mild increase then continues with the elapse of time. The rapidly increased absorbance is measured via the first measurement, and the mildly increased absorbance with the elapse of time is measured via the second measurement. The measured value attained via the first measurement reflects the amount of cholesterol in HDL, and the difference between the second measurement and the first measurement indicates the amount of cholesterol in LDL.
(4) Assay in the triglyceride assay system
   LDL triglyceride and total triglyceride, HDL triglyceride and total triglyceride, or LDL triglyceride and HDL triglyceride are simultaneously assayed in the same manner as in (1) to (3) above, except that the enzymes used therein are changed from cholesterol esterase and cholesterol oxidase to triglyceride lipase, glycerol kinase, and glycerol-3-phosphate oxidase. In this assay system, cholesterol esterase may be added to the reaction system, in order to accelerate the enzyme reaction.
   As shown in Fig. 1, in the second step, the absorbance rapidly increases simultaneously with the initiation of the second step, and a mild increase then continues with the elapse of time. The rapidly increased absorbance is measured via the first measurement, and the mildly increased absorbance with the elapse of time is measured via the second measurement. The measured value attained via the second measurement reflects the amount of total glyceride or LDL glyceride, and the difference between the second measurement and the first measurement indicates the amount of LDL glyceride or HDL glyceride.
(5) Assay in creatinine, uric acid, or glucose assay system
   (i) As methods for assaying creatinine, a creatinine deaminase method and a creatinine amidohydrolase (creatininase) method are available. In the former method, an enzyme is allowed to act on creatinine to generate ammonia, and the resulting ammonia is measured via colorimetry. In the latter method, creatinine is converted into creatine by the action of creatininase, sarcosine is generated with the aid of creatinase, and hydrogen peroxide is generated with the aid of sarcosine oxidase. Subsequently, quinone pigments generated from various chromogens in the presence of peroxidase are quantified.
   (ii) As a method for assaying glucose, a GOD-POD method that utilizes a GOD reaction, wherein glucose oxidase (GOD) is allowed to act on glucose, O₂, and H₂O to generate glucuronic acid and hydrogen peroxide is available. The GOD reaction is allowed to proceed in the presence of peroxidase (POD) to measure oxidation, color development, and the like of a pigment.
      There are an aminoantipyrine-phenol assay system wherein 4-aminoantipyrine (4AA) and phenol are subjected to oxidation condensation with the aid of H₂O₂ generated in the GOD reaction in the presence of POD and a resulting quinone pigment (absorption maximum: 505 nm) is subjected to colorimetry and an MBTH-DMA assay system wherein 3-methyl-2-benzothiazolinehydrazone and dimethylaniline are subjected to oxidation condensation with the aid of H₂O₂ generated in the GOD reaction in the presence of POD and a resulting indamine pigment (absorption maximum: 600 nm) is subjected to colorimetry.
   (iii) As a method for assaying uric acid, an uricase-peroxidase method is available. In this method, uricase is allowed to act on uric acid to generate H₂O₂, the resulting H₂O₂, N-ethyl-2-hydroxy-N-toluidine (EHSPT), and 4-aminoantipyrine (4AA) are subjected to oxidation condensation in the presence of peroxidase to generate a reddish-purple quinone pigment, and the quinone pigment is measured at 546 nm to determine an uric acid content.

   When the first target analyte is glucose and the second target analyte is uric acid in two target analytes, for example, the first reagent is comprised of GOD and then with either 4-aminoantipyrine or a phenol or aniline hydrogen donor compound, which is a reagent composition associated with generation of a quinone pigment. Also, the second reagent is comprised of uricase and then with a reagent composition that is not included in the first reagent, i.e., either of 4-aminoantipyrine, a phenol or aniline hydrogen donor compound, or peroxidase. In such a case, the first reaction intermediate and the second reaction intermediate are each hydrogen peroxide, and the first reaction product and the second reaction product are each a quinone pigment.
   When the first target analyte is glucose and the second target analyte is creatinine in two target analytes, for example, the first reagent is comprised of GOD and then with either 4-aminoantipyrine or a phenol or aniline hydrogen donor compound, which is a reagent composition associated with generation of a quinone pigment. Also, the second reagent is comprised of creatininase, creatinase, and sarcosine oxidase and then with a reagent composition that is not included in the first reagent, i.e., either of 4-aminoantipyrine, a phenol or aniline hydrogen donor compound, or peroxidase. In such a case the first reaction intermediate is hydrogen peroxide, the first reaction product is a quinone pigment, the second reaction intermediate is sarcosine, and the second reaction product is a quinone pigment. The second reagent may be comprised of either of 4-aminoantipyrine, a phenol or aniline hydrogen donor compound, or peroxidase, which is not included in the first reagent and with creatinine deaminase. In such a case, the first reaction intermediate is hydrogen peroxide, the first reaction product is a quinone pigment, and the second reaction product is ammonia.
(6) When assay is carried out in, for example, the glucose, glutamate oxaloacetate transaminase (GOT) (aspartate aminotransferase (AST)), glutamate pyruvate transaminase (GPT) (alanine aminotransferase (ALT)), lactate dehydrogenase (LDH), or creatine phosphokinase (CPK) system, the target analytes are allowed to react in the presence of NAD (oxidized nicotinamide adenine dinucleotide) and/or NADH (reduced nicotinamide adenine dinucleotide), the target analytes can be quantified by measuring at least one of the amount of NAD and/or NADH increased, the amount thereof decreased, the rate of increase, and the rate of decrease resulting from the reaction. Specific examples of assay systems are as follows.
   (i) A glucose assay system: Glucose is converted into glucose 6-phosphate by the action of hexokinase (HK), and the resulting glucose 6-phosphate is converted into gluconolactone 6-phosphate and NADH in the presence of NAD and glucose 6-phosphate dehydrogenase (G6PDH). Upon reduction of NAD to NADH, the absorbance at 340 nm is increased, and the activity value is then determined by measuring the amount or rate of such increase.
   (ii) A GOT assay system: L-aspartic acid and α-ketoglutaric acid are converted into glutamic acid and oxalacetic acid by the action of GOT, the resulting oxalacetic acid is further converted into malic acid and NAD in the presence of NADH (reduced nicotinamide adenine dinucleotide) and MDH (malate dehydrogenase). Upon oxidation of NADH to NAD, the absorbance at 340 nm is decreased, and the activity value is then determined by measuring the amount or rate of such decrease.
   (iii) A GPT assay system: L-alanine and α-ketoglutaric acid are converted into glutamic acid and pyruvic acid by the action of GPT, and pyruvic acid is converted into lactic acid in the presence of NADH with the aid of lactate dehydrogenase. Upon oxidation of NADH to NAD, the absorbance at 340 nm is decreased, and the activity value is then determined by measuring the amount or rate of such decrease.
   (iv) An LDH assay system: Pyruvic acid is converted into lactic acid in the presence of NADH with the aid of LDH. Upon oxidation of NADH to NAD, the absorbance at 340 nm is decreased, and the activity value is then determined by measuring the amount or rate of such decrease.
   (v) A CPK assay system: Creatine and adenosine-5'-triphosphate (ATP) are converted into creatine phosphate and adenosine-5'-diphosphate (ADP) with the aid of CPK, the ADP is converted into ATP and pyruvic acid (Pyr) in the presence of phosphenol pyruvate (PEP) by the action of pyruvic acid kinase (PK), and the pyruvic acid is converted into lactic acid and NAD in the presence of NADH by the action of lactate dehydrogenase. Upon oxidation of NADH into NAD, the absorbance at 340 nm is decreased, and the activity value is then determined by measuring the amount or rate of such decrease.

   Any other target analytes can be assayed, provided that such target analytes are allowed to react in the presence of NAD and/or NADH (reduced nicotinamide adenine dinucleotide), and increase/decrease of NAD and/or NADH resulting from the reaction can be assayed.
   When the first target analyte is glucose and the second target analyte is GOT in two target analytes, for example, the first reagent is comprised of HK and ATP, with at least one of L-aspartic acid or α-ketoglutaric acid, and either one of NAD or G6PDH. The first reagent may be optionally comprised of NADH. Also, the second reagent may be comprised of a substance that is not included in the first reagent among MDH, L-aspartic acid, α-ketoglutaric acid, NAD, and G6PDH.
   When the first target analyte is glucose and the second target analyte is GPT in two target analytes, for example, the first reagent is comprised of HK and ATP, at least one of L-alanine or α-ketoglutaric acid, either one of NAD or G6PDH. The first reagent may be optionally comprised of NADH. Also, the second reagent may be comprised of a substance that was not included in the first reagent, among LDH, L-alanine, α-ketoglutaric acid, NAD, and G6PDH.
   When the first target analyte is glucose and the second target analyte is LDH in two target analytes, for example, the first reagent is comprised of HK and ATP and then with either NAD or G6PDH. The first reagent may be optionally comprised of NADH. The second reagent may be comprised of pyruvic acid and LDH and then with either NAD or G6PDH, which was not included in the first reagent.
   When the first target analyte is glucose and the second target analyte is CPK in two target analytes, for example, the first reagent is comprised of HK and ATP, then with at least one of creatine, PEP, and PK, and further with either NAD or G6PDH. The first reagent may be optionally comprised of NADH. The second reagent may be comprised of LDH, creatine, PEP, or PK, which were not included in the first reagent, then with LDH, and then with NAD or G6PDH, which was not included in the first reagent.
(7) When assay is carried out in an amylase (AMY) or alkaline phosphatase (ALP) system, for example, the target analytes are allowed to act on a substrate to generate p-nitrophenol (PNP), and the rate of generation thereof is measured. Thus, the target analytes can be quantified. In the case of the γ-GTP assay system, 5-amino-2-nitrobenzoate is generated, and the rate of generation thereof is measured. Thus, the target analytes can be quantified. Such PNP and 5-amino-2-nitrobenzoate can be measured in terms of the absorbance at the same wavelength, i.e., 405 nm. Specific examples of assay systems are as follows.
   (i) AMY assay system: AMY is allowed to act on 4,6-ethylidene-4-nitrophenyl-α-(1→4)-D-maltoheptaoside (Et.G₇-pNP) to generate G2PNP, G3PNP, and G4PNP by the action of α-amylase. A coupling enzyme, α-glucosidase (α-GH), is allowed to act thereon to release PNP, and the rate of the absorbance resulting from the generation of PNP is measured. Thus, the amylase activity can be determined.
   (ii) ALP assay system: ALP is allowed to act on p-nitrophenyl phosphate to release p-nitrophenol. The rate of PNP generation is measured in terms of the absorbance at 405 nm to determine the alkaline phosphatase activity in the blood serum.
   (iii) γ-GTP assay system: γ-GTP is allowed to act on L-γ-glutamyl-3-carboxy-4-nitroanilide and glycylglycine to generate 5-amino-2-nitrobenzoate, and the rate of generation of 5-amino-2-nitrobenzoate is measured in terms of the absorbance at 405 nm to determine the activity value of γ-GTP.

Any other target analytes can be assayed, provided that generation of PNP or 5-amino-2-nitrobenzoate can be measured by reacting such target analytes.

When the first target analyte is AMY and the second target analyte is ALP in two target analytes, for example, the first reagent may be comprised of Et-G₇-pNP, and the second reagent may becomprised ofα-glucosidase (α-GH) and p-nitrophenyl phosphate.

The present invention relates to a method for simultaneously quantifying two target analytes, such as two lipid components, in the analyte sample. The present invention includes a kit for performing a method for simultaneously quantifying two target analytes, which comprises the first step of treating the first target analyte in a biological sample to generate hydrogen peroxide and the second step of converting hydrogen peroxide obtained in the first step into a quinone pigment and treating the second target analyte to convert the resulting hydrogen peroxide into a quinone pigment, wherein a quinone pigment is not generated in the first step and two measurements, i.e., the first measurement of a quinone pigment originating from the first target analyte and the second measurement of quinone pigments originating from the first target analyte and the second target analyte, are carried out in the second step, thereby simultaneously quantifying two target analytes based on the amounts of generated quinone pigments. The kit of the present invention comprises the first reagent and the second reagent. The first reagent may further comprise a reagent composition associated with generation of a quinone pigment, such as peroxidase, 4-aminoantipyrine, and a phenol or aniline hydrogen donor compound, although it does not comprise all of the peroxidase, 4-aminoantipyrine, and a phenol or aniline hydrogen donor compound simultaneously. The first reagent does not contain peroxidase but contains either 4-aminoantipyrine or a phenol or aniline hydrogen donor compound. The first reagent may comprise an adequate buffer, albumin, and the like. The second reagent at least comprises a reagent composition associated with generation of a quinone pigment, which is not included in the first reagent, i.e., peroxidase, 4-aminoantipyrine, or a phenol or aniline hydrogen donor compound. The second reagent may further comprise an adequate buffer, albumin, and the like. Further, the first reagent comprises a surfactant and an enzyme that act on the first target analyte, and the second reagent may comprise a surfactant that acts at least on the second target analyte. The absorbance of the colored quinone resulting from the reaction of such reagent composition can be measured. The kit of the present invention further comprises a standard solution of the target analyte with known concentrations and a buffer.

### Examples

Hereafter, the present invention is described in greater detail with reference to the following examples, although the technical scope of the present invention is not limited thereto.

### Example 1

Concerning reagents used for simultaneously assaying LDL cholesterol and total cholesterol in a cholesterol assay system, the compositions of the reagent compositions used in the first step and in the second step (i.e., the first reagent composition and the second reagent composition) were adjusted as shown below. A reagent comprising all compositions associated with color development in the first reagent was used in a control assay system 1, and the reagent of the present invention was used in the assay system 2.

| Assay system 1 | |
|---|---|
| First reagent composition | |
| PIPES buffer (pH 7.0) | 50 mmol/l |
| TOOS | 0.7 mmol/l |
| 4-aminoantipyrine | 1.5 mmol/l |
| Cholesterol esterase | 0.8 IU/ml |
| Cholesterol oxidase | 0.5 IU/ml |
| Surfactant, Emulgen B-66 (Kao Corporation) | 0.2 % |
| POD (peroxidase) | 1.0 IU/ml |
| Magnesium chloride | 10 mmol/l |

| Second reagent composition | |
|---|---|
| PIPES buffer (pH 7.0) | 50 mmol/l |
| Triton X100 | 3.0 % |

| Assay system 2 | |
|---|---|
| First reagent composition | |
| PIPES buffer (pH 7.0) | 50 mmol/l |
| TOOS | 2 mmol/l |
| Cholesterol esterase | 0.6 IU/ml |
| Cholesterol oxidase | 0.5 IU/ml |
| Surfactant, Emulgen B-66 (Kao Corporation) | 0.27 % |

| Second reagent composition | |
|---|---|
| PIPES buffer (pH 7.0) | 50 mmol/l |
| Surfactant, Polidocanol (Thesit) | 1 % |

| (Roche Diagnostics) | |
|---|---|
| 4-Aminoantipyrine | 4 mmol/l |
| POD | 6.5 IU/ml |

The above reagents were stored at 8°C for a given period of time, and variations in the absorption spectra of the first reagent were measured.

The absorption spectra were measured using a spectrophotometer U-3000 (Hitachi, Ltd.).

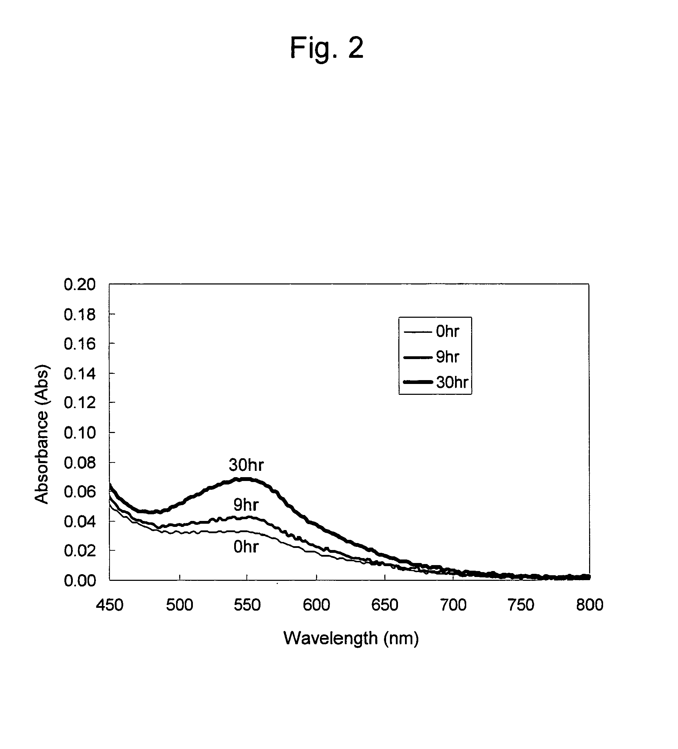

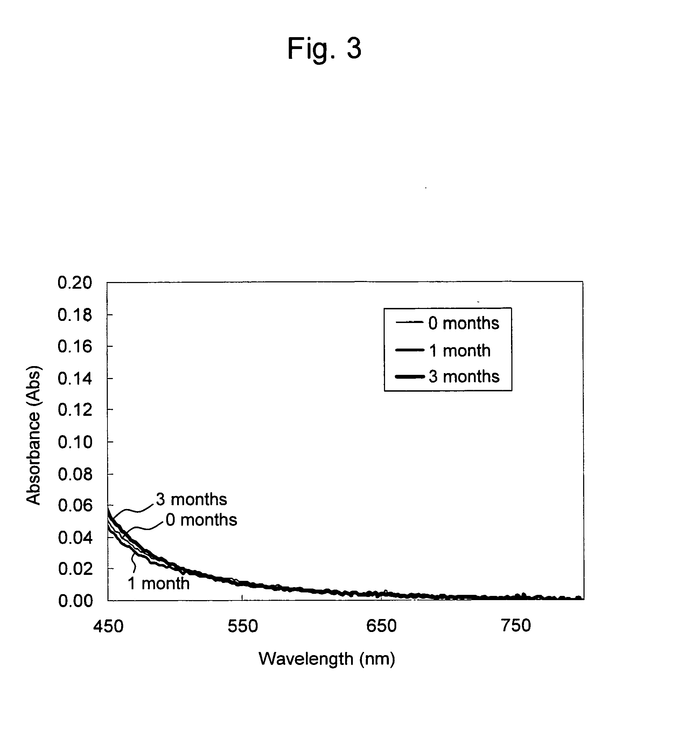

Fig. 2 and Fig. 3 show the results of assaying the absorption spectra of the product of the first reagent.

As shown in Fig. 2, the first reagent spontaneously develops color in the assay system 1 in which color-developing agents are collectively present in the first reagent, and variations in the absorption spectra are observed within several hours. In the case of the present invention wherein compositions associated with color development are present in the first reagent and in the second reagent as shown in Fig. 3, substantially no variation is observed in the absorption spectra after several months. This indicates that the reagent can remain stable in a liquid state according to the present invention.

### Example 2

The reagent similar to that used in Example 1 was used to inspect variations in the calibration absorption spectra caused by the storage of the reagent.

An automatic analyzer, TBA-30R (Toshiba Corporation), was used.

### (Reagent for simultaneous analysis of LDL-C and T-CHO)

### Assay conditions: Automatic analysis of multiple items

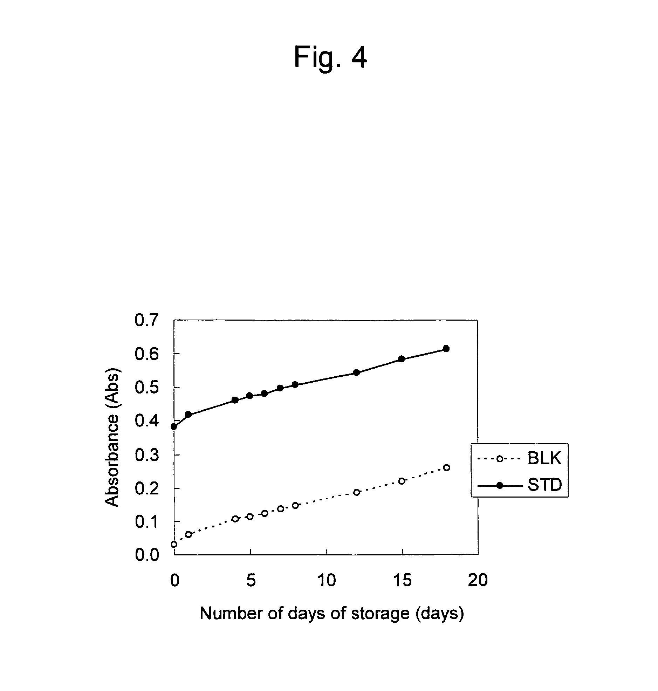

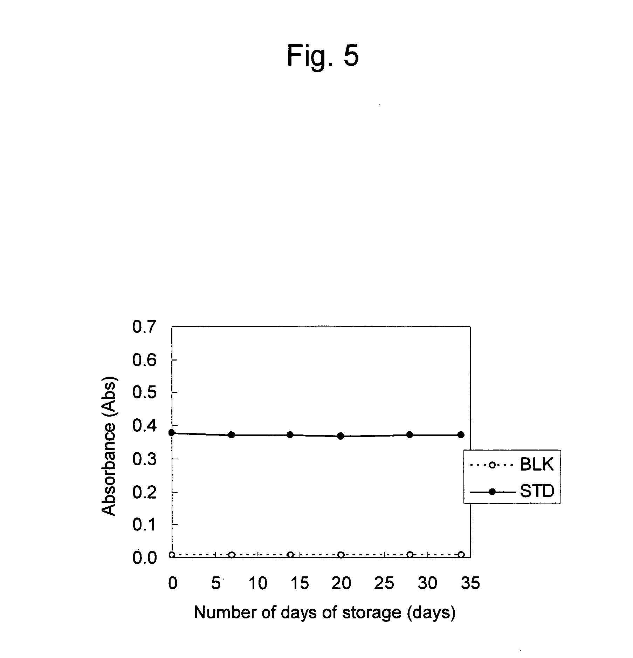

The first reagent (300 µl) preheated to 37°C was mixed with 4 µl each of reagents, the reaction was allowed to proceed at 37°C for 5 minutes, 100 µl of the second reagent was added, the reaction was allowed to proceed for an additional 5 minutes, and the absorbance at 600 nm was assayed. LDL cholesterol was assayed based on the differences in the absorbance between 30 seconds and 5 minutes after the addition of the second reagent. Total cholesterol was assayed based on the extent of change in the absorbance after the addition of the second reagent. The results are shown in Fig. 4 and in Fig. 5.

As shown in Fig. 4 and in Fig. 5, the calibration absorption spectra vary within a short period of time along with spontaneous color development of the reagent in assay system 1, and, thus, stable properties cannot be realized. In the present invention, however, the calibration absorption spectra remain stable for a long period of time. This indicates that properties of the reagent can be maintained.

### Example 3

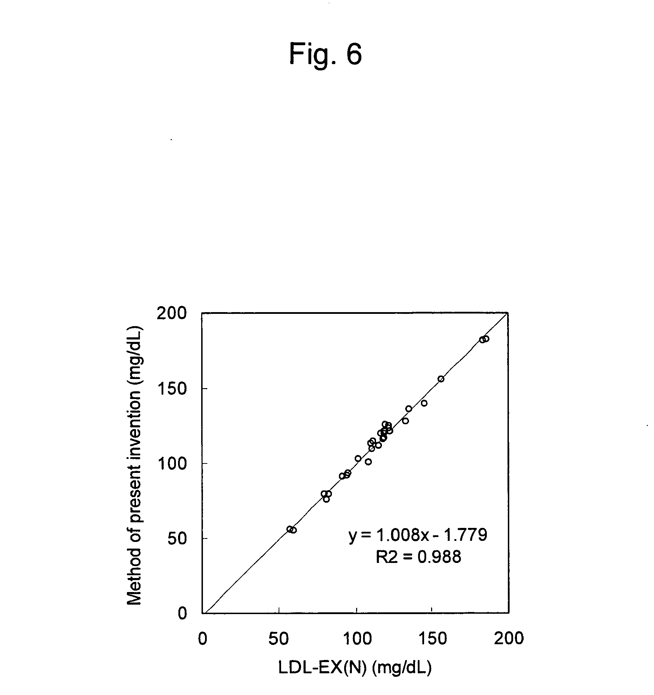

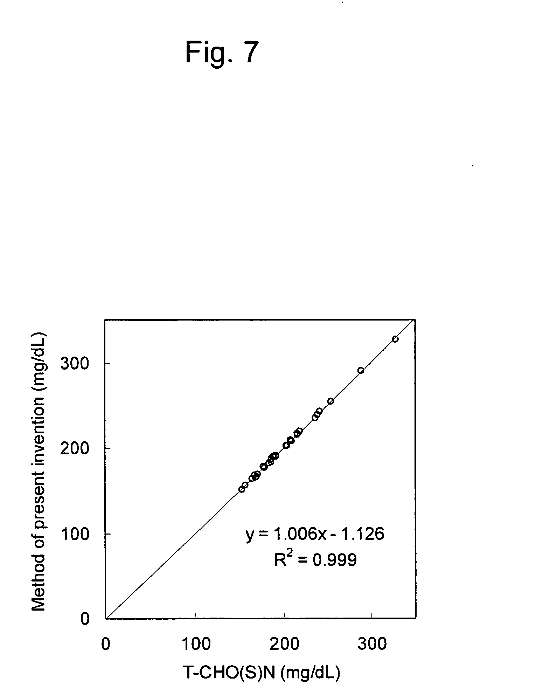

A reagent similar to that used in Example 1 was prepared and 30 human serum samples were assayed. LDL-EX N (Denka Seiken Co., Ltd.) was used as the evaluation-target product of LDL cholesterol, and T-CHO(S)N (Denka Seiken Co., Ltd.) was used as the evaluation-target product of total cholesterol. Fig. 6 shows the correlation between the LDL cholesterol value assayed with the use of LDL-EX N, which is a evaluation-target product, and the value assayed in accordance with the method of the present invention. Fig. 7 shows the correlation between the total cholesterol value assayed with the use of T-CHO(S)N, which is the evaluation-target product, and the value assayed in accordance with the method of the present invention. As shown in Fig. 6 and in Fig. 7, the method for simultaneous quantification of the present invention produces similar results as in the case of independent assay of LDL cholesterol and total cholesterol.

### Example 4

As reagents for simultaneously assaying HDL cholesterol and total cholesterol in a cholesterol assay system, the composition of the reagent compositions used in the first step and the second step (i.e., the first reagent composition and the second reagent composition) were adjusted as shown below.

| First reagent composition | |
|---|---|
| PIPES buffer (pH 7.0) | 100 mmol/l |
| HDAOS | 0.6 mmol/l |
| Cholesterol esterase | 1.4 IU/ml |
| Cholesterol oxidase | 0.6 IU/ml |
| Surfactant, Pluronic F-68 (Asahi Denka Co., Ltd.) | 0.25 % |

| Second reagent composition | |
|---|---|
| PIPES buffer (pH 7.0) | 100 mmol/l |
| Surfactant, Emulgen B-66 (Kao Corporation) | 1.4% |
| 4-aminoantipyrine | 4 mmol/l |
| POD | 3.5 IU/ml |

With the use of the obtained reagents, 30 human serum samples were assayed. HDL-EX (Denka Seiken Co., Ltd.) was used as the evaluation-target product of HDL cholesterol, and T-CHO (S)N (Denka Seiken Co., Ltd.) was used as the control of total cholesterol. Table 1 shows the correlation between the HDL cholesterol value assayed with the use of HDL-EX N, which is the evaluation-target product, and the value assayed in accordance with the method of the present invention. Table 2 shows the correlation between the total cholesterol value assayed with the use of T-CHO(S)N, which is the evaluation-target product, and the value assayed in accordance with the method of the present invention. As shown in Table 1 and in Table 2, the method for simultaneous quantification of the present invention produces similar results as in the case of independent assay of HDL cholesterol and total cholesterol. Table 1 shows the HDL cholesterol values assayed with the use of the reagent for simultaneously assaying HDL cholesterol and total cholesterol according to the present invention and the HDL cholesterol values independently assayed. Table 2 shows the correlation between the total cholesterol values assayed with the use of the reagent for simultaneously assaying HDL cholesterol and total cholesterol according to the present invention and total cholesterol values independently assayed.

**Table 1**

| | Independent assay | Simultaneous assay |
|---|---|---|
| Analyte 1 | 68.1 | 67.7 |
| Analyte 2 | 72.9 | 72.6 |
| Analyte 3 | 48.4 | 46.6 |
| Analyte 4 | 59.1 | 57.7 |

**Table 2**

| | Independent assay | Simultaneous assay |
|---|---|---|
| Analyte 1 | 212.9 | 221.6 |
| Analyte 2 | 257.3 | 251.8 |
| Analyte 3 | 153.8 | 147.1 |
| Analyte 4 | 179.8 | 187.0 |

All publications, patents, and patent applications cited herein are incorporated herein by reference in their entirety.

## Claims

1. A method for simultaneously quantifying two target analytes in a sample using two types of reagents, wherein the reaction product associated with the first target analyte is detected at an early stage of the second step and then the reaction product associated with the second target analyte is detected.

2. The method for simultaneously quantifying two target analytes in a sample using two types of reagents according to claim 1, wherein the first target analyte reacts with a reagent composition contained in the first reagent to generate the first reaction intermediate, the second target analyte reacts with a reagent composition contained in the second reagent to generate the second reaction intermediate, the first reaction intermediate reacts with the reagent composition contained in the second reagent to generate the optically measurable first reaction product, the second reaction intermediate reacts with the reagent composition contained in the second reagent to generate the optically measurable second reaction product, the method comprising the first step of adding the first reagent to the sample to treat the first target analyte and generate the first reaction intermediate and the second step of adding the second reagent to generate the first reaction product from the first reaction intermediate obtained in the first step and treating the second target analyte to generate the second reaction product from the resulting second reaction intermediate, wherein, in the first step, the first reaction product is not generated and, in the second step, measurement is carried out twice, i.e., the first reaction product originating from the first target analyte is measured in the first measurement, and the second reaction product originating from the second target analyte or the first reaction product originating from the first target analyte, and the second reaction product originating from the second target analyte are measured in the second measurement, thereby simultaneously quantifying two target analytes based on the amounts of the resulting first and second reaction products.

3. The method according to claim 1 or 2, wherein the first reaction product originating from the first target analyte and the second reaction product originating from the second target analyte have the same absorption wavelengths.

4. The method according to claim 3, wherein the first reaction product originating from the first target analyte and the second reaction product originating from the second target analyte are the same substances.

5. The method according to any one of claims 1 to 4, wherein the first reaction intermediate and the second reaction intermediate are the same substances.

6. The method according to any one of claims 1 to 5, wherein the first target analyte and the second target analyte are selected from the group consisting of lipid components in the analyte sample, i.e., creatinine, uric acid, glucose, glutamate oxaloacetate transaminase (GOT) (aspartate aminotransferase (AST)), glutamate pyruvate transaminase (GPT) (alanine aminotransferase (ALT)), γ-glutamyl transpeptidase (γ-GTP), lactate dehydrogenase (LDH), alkaline phosphatase (ALP), creatine phosphokinase (CPK), and amylase (AMY).

7. The method according to claim 6, wherein the first target analyte and the second target analyte are lipid components in the analyte sample.

8. The method according to claim 7, wherein the lipid components are cholesterol or triglyceride components in the lipoprotein.

9. The method according to any one of claims 1 to 8, wherein the first and the second reaction products originating from the first and the second target analytes and the first and the second reaction products originating from the first and the second reaction intermediates are generated by oxidation-reduction reaction.

10. The method for simultaneously quantifying two lipid components in a sample using two types of reagents according to any one of claims 1 to 9, which comprises the first step of treating the first target analyte in a biological sample to generate hydrogen peroxide and the second step of converting hydrogen peroxide obtained in the first step into a quinone pigment and treating the second target analyte to convert the resulting hydrogen peroxide into a quinone pigment, wherein a quinone pigment is not generated in the first step, and the second step comprises two measurements of the first measurement of a quinone pigment originating from the first target analyte and the second measurement of quinone pigments originating from the first target analyte and the second target analyte, thereby simultaneously quantifying two target analytes based on the amounts of the generated quinone pigments.

11. The method according to claim 10, wherein the reagent composition associated with generation of a quinone pigment comprises 4-aminoantipyrine, a phenol or aniline hydrogen donor compound, and peroxidase and the first step involves the addition of either a 4-aminoantipyrine or a phenol or aniline hydrogen donor compound and the second step involves the addition of a reagent composition that was not added in the first step.

12. The method according to claim 10 or 11, wherein the assay of the lipid component is an assay of a cholesterol or triglyceride component in the lipoprotein.

13. The method according to any one of claims 10 to 12, wherein, when the lipid component is cholesterol, the two target analytes are total cholesterol and low-density lipoprotein (hereafter referred to as "LDL") cholesterol, total cholesterol and high-density lipoprotein (hereafter referred to as "HDL") cholesterol, or LDL cholesterol and HDL cholesterol in the blood.

14. The method according to any one of claims 10 to 12, wherein, when the lipid component is triglyceride, two target analytes are total triglyceride and LDL triglyceride, total triglyceride and HDL triglyceride, or LDL triglyceride and HDL triglyceride in the blood.

15. The method according to any one of claims 10 to 14, wherein, when the two target analytes are total lipid components and a lipid component in HDL or LDL, the method comprises the first step of generating hydrogen peroxide in the presence of a reagent comprising an enzyme and a surfactant that selectively act on lipoproteins other than HDL or LDL and the second step of converting hydrogen peroxide obtained in the first step into a quinone pigment and generating hydrogen peroxide in the presence of a reagent that selectively reacts with HDL or LDL to convert hydrogen peroxide into a quinone pigment.

16. The method according to any one of claims 10 to 14, which, when the two target analytes are lipid components in HDL and LDL, comprises the first step of generating hydrogen peroxide in the presence of a reagent comprising a given enzyme and surfactant that selectively act on HDL and the second step of converting hydrogen peroxide obtained in the first step into a quinone pigment and generating hydrogen peroxide in the presence of a reagent that selectively reacts with LDL to convert hydrogen peroxide into a quinone pigment.

17. The method according to any one of claims 1 to 16, wherein, changes in the absorbance in the second step indicate a two-phase increase comprising a rapid increase in the absorbance immediately after the addition of the second reagent and a mild increase thereafter, and a target analyte is quantified based on the amount of the latter mild change in the absorbance.

18. The method according to any one of claims 1 to 17, wherein the total cholesterol is quantified based on the total extent of change in the absorbance in the second step.

19. The method according to any one of claims 1 to 18, wherein the analysis is carried out using an automated clinical biochemical analyzer under different assay parameters via a single assay operation.

20. The method according to any one of claims 1 to 19, wherein the first and the second steps each comprise the addition of respective liquid reagents.

21. A method for stabilizing a liquid reagent in the method according to any one of claims 1 to 20, wherein the first reagent that is added in the first step comprises a reagent composition associated with generation of a quinone pigment, i.e., any of 4-aminoantipyrine and a phenol or aniline hydrogen donor compound, and the second reagent comprises a substance that is not contained in the first reagent selected from among 4-aminoantipyrine, a phenol or aniline hydrogen donor compound, and peroxidase.

22. A kit for performing a method for simultaneously quantifying two lipid components in a sample using two types of reagents, the method comprising the first step of treating the first target analyte in a biological sample to generate hydrogen peroxide and the second step of converting hydrogen peroxide obtained in the first step into a quinone pigment and treating the second target analyte to convert the resulting hydrogen peroxide into a quinone pigment, wherein a quinone pigment is not generated in the first step and the second step comprises two measure operations of the first assay of a quinone pigment originating from the first target analyte and the second assay of quinone pigments originating from the first target analyte and the second target analyte, thereby simultaneously quantifying two target analytes based on the amounts of generated quinone pigments, and wherein the first reagent comprises a reagent composition associated with generation of a quinone pigment, i.e., any of 4-aminoantipyrine and a phenol or aniline hydrogen donor compound, and the second reagent comprises a substance that is not contained in the first reagent selected from among 4-aminoantipyrine, a phenol or aniline hydrogen donor compound, and peroxidase.

23. The kit according to claim 22, wherein the first reagent comprises a surfactant and an enzyme that act on the first target analyte, and the second reagent at least comprises a surfactant that acts on the second target analyte.
